(19) Europäisches Patentamt
European Patent Office
Office européen des brevets

(11) Veröffentlichungsnummer: **0 553 384 B1**

# (12) EUROPÄISCHE PATENTSCHRIFT

(45) Veröffentlichungstag der Patentschrift: **29.03.95**

(51) Int. Cl.[6]: **C07C 229/22**

(21) Anmeldenummer: **92101617.6**

(22) Anmeldetag: **31.01.92**

## (54) Acetylsalicyloyl-Carnitin und Verfahren zu seiner Herstellung.

(43) Veröffentlichungstag der Anmeldung:
**04.08.93 Patentblatt 93/31**

(45) Bekanntmachung des Hinweises auf die Patenterteilung:
**29.03.95 Patentblatt 95/13**

(84) Benannte Vertragsstaaten:
**AT BE DE ES FR GB IT LU NL PT SE**

(56) Entgegenhaltungen:
**EP-A- 0 150 688**
**CH-A- 389 641**
**CH-A- 679 395**

(73) Patentinhaber: **LONZA AG**
**(Geschäftsleitung: 4002 Basel)**
**CH-3945 Gampel/Wallis (CH)**

(72) Erfinder: **Meul, Thomas, Dr.**
**Meisenweg 1**
**CH-Visp (Kanton Wallis) (CH)**

(74) Vertreter: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P.**
**Barz**
**Siegfriedstrasse 8**
**D-80803 München (DE)**

EP 0 553 384 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft 3-(2-Acetoxybenzoyloxy)-4-(trimethylammonio)-buttersäurebetain, Acetylsalicyloylcarnitin,der allgemeinen Formel

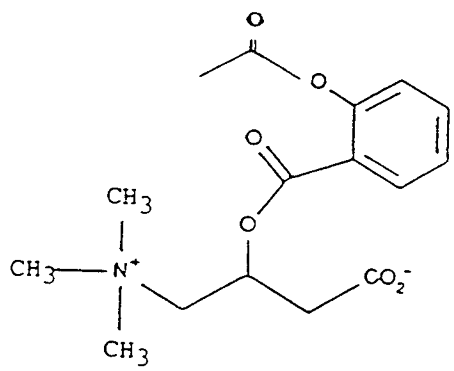

I

in racemischer und optisch aktiver Form, dessen pharmazeutisch akzeptable Salze sowie ein Verfahren zu dessen Herstellung. 3-(2-Acetoxybenzoyloxy)-4-(trimethylammonio)-buttersäurebetain ist als Ester der Acetylsalicylsäure mit Carnitin (Acetylsalicyloylcarnitin) ein Salicylsäurederivat mit vielversprechenden therapeutischen Eigenschaften.

Salicylsäure wird in Form ihres Acetylderivats in grossem Umfang als Analgetikum eingesetzt. Obwohl dieses Acetylderivat (bekannt u.a. als Aspirin$^{R}$) ursprünglich entwickelt wurde, um störende Nebenwirkungen der schon früher bekannten Salicylsäure zu verringern, ist es dennoch mit einigen Eigenschaften behaftet, die seine Anwendungsmöglichkeiten einschränken. Zu diesen ungünstigen Eigenschaften zählt vor allem seine geringe Wasserlöslichkeit, insbesondere in saurem Milieu, also beispielsweise im Magensaft. Die geringe Löslichkeit kann bei oraler Verabreichung von wässrigen Lösungen zum Ausfallen des Wirkstoffs im Magen führen. Dieser Effekt ist nicht nur bei Personen mit empfindlicher oder vorgeschädigter Magenschleimhaut unerwünscht, weil er bei diesen Personen zu ernsthaften Nebenwirkungen führen kann, sondern er verzögert ganz allgemein die Resorption und damit auch den Eintritt der analgetischen Wirkung. Ausserdem kann Acetylsalicylsäure praktisch nur oral, aber nicht parenteral, also beispielsweise intravenös oder intraperitoneal, oder topisch appliziert werden. Gerade wegen des schnellen Wirkungseintritts und/oder der Schonung des Gastrointestinaltrakts wäre aber eine parenterale Applikation oft wünschenswert.

Aufgabe der vorliegenden Erfindung war daher, ein Salicylsäurederivat zur Verfügung zu stellen, das auch im sauren Bereich gut wasserlöslich ist, leicht resorbiert wird, eine möglichst geringe Toxizität aufweist und sowohl oral als auch parenteral oder topisch appliziert werden kann und in allen Darreichungsformen eine rasch einsetzende analgetische Wirkung zeigt. Erfindungsgemäss wird diese Aufgabe durch das 3-(2-Acetoxybenzoyloxy)-4-(trimethylammonio)-buttersäurebetain nach Patentanspruch 1 gelöst. Die Verbindung weist ein asymmetrisches Kohlenstoffatom auf und kann daher in zwei spiegelbildlichen optisch aktiven Formen und als racemisches Gemisch auftreten. Bevorzugt ist dabei das Enantiomer mit (R)-Konfiguration, das sich von dem natürlich vorkommenden L-Carnitin ableitet.

Die günstigen physikalisch-chemischen Eigenschaften, wie hohe Wasserlöslichkeit und günstiger pH-Wert der Lösung, werden allerdings auch von dem (S)-Enantiomer und dem Racemat erreicht.

Untersuchungen an Ratten zeigten bereits eine äusserst geringe akute Toxizität von Acetylsalicyloyl-L-carnitin. Dosen bis zu 1000 mg/kg Körpergewicht wurden oral ohne weiteres vertragen und bei intravenöser und intraperitonealer Verabreichung therapeutischer Mengen wurden keine schädlichen Nebenwirkungen beobachtet.

Selbstverständlich liegt es ebenfalls im Rahmen der Erfindung, Salze des Acetylsalicyloylcarnitins mit pharmazeutisch akzeptablen Säuren zu bilden und die Verbindung in dieser Form zu verwenden.

Das Acetylsalicyloylcarnitin wird erfindungsgemäss durch Acetylierung des Salicyloylcarnitins hergestellt. Salicyloylcarnitin kann durch Umsetzung eines 2-Methoxybenzoylhalogenids mit einem Carnitinhydrohalogenid, anschließende Demethylierung und Deprotonierung zum Betain erhalten werden.

Als Acetylierungsmittel eignet sich besonders Acetylchlorid oder Essigsäureanhydrid in Gegenwart katalytischer Mengen $H_2SO_4$.

Die Acetylierung erfolgt zweckmässig in einem Temperaturbereich von 40 °C und 100 °C in Gegenwart von Essigsäure als Lösungsmittel.

Erfahrungsgemäss kann nach einer Reaktionszeit von ca. 5 h nach üblicher Aufarbeitung das Acetylsalicyloylcarnitin in guter Ausbeute und grosser Reinheit gewonnen werden.
Ebenso liegt es im Rahmen des erfindungsgemässen Verfahrens, anschliessend das Betain durch Zugabe einer pharmazeutisch akzeptablen Säure in ein entsprechendes Salz überzuführen.

Das nachfolgende Beispiel verdeutlicht die Durchführung des erfindungsgemässen Herstellungsverfahrens.

Beispiel

Acetylsalicyloyl-L-Carnitin•HCl

0,95 g Salicoyl-L-Carnitin-HCl ($[\alpha_D^{20}$ = -31,2° (c=1, $H_2O$); Smp.: 185-187°C) wurden mit 2,35 g Acetylchlorid und 5,0 ml Essigsäure gemischt und 5 h auf 60°C erhitzt. Anschliessend wurde die Reaktionsmischung im Vakuum eingedampft und der Rückstand mit 10,0 ml Essigester aufgeschlämmt. Das kristallisierte Produkt wurde mit 5,0 ml Essigester gewaschen und im Vakuum bei 40°C getrocknet. Man erhielt 0,95g weisses, kristallines Acetylsalicyloyl-L-Carnitin•HCL vom Smp. 154-158°C.

$^1$H-NMR (DMSO-$d_6$, 300 MHz) $\delta$=12,9 (br.s, 1H), 8,04 (d, 1H, J=1,9 Hz), 7,73 (t, 1H, J=7,5 Hz), 7,46 (t, 1H, J=7,6 Hz), 7,28 (d, 1H, J=8,0 Hz), 5,70 (m, 1H), 4,08-3,83 (m, 2H), 3,20 (s, 9H), 2,93-2,78 (m, 2H), 2,33 (s, 3H);

$[\alpha]_D^{20}$ = -41,7° (c=1, $H_2O$).

Magenverträglichkeitstest an Ratten (Ulcer Index)

Das R-(-)-3-(2-Acetoxybenzoyloxy)-4-(trimethylammonio)-buttersäurebetain•HCl (Acetylsalicoyl-L-Carnitin = ASC) wurde im Vergleich zu Acetylsalicylsäure (ASA) an männlichen Ratten getestet, indem anlehnend an die Methode von Okabe et al., Japan. J. Pharmacol. 1974, 24, 363ff, Magenschleimhautveränderungen induziert wurden.
Die Testsubstanzen wurden in einer 1% Carboxymethylcellulose-Suspension (1% CMC) den Testratten p.o. verabreicht.
Die Magenschleimhautveränderungen wurden mittels dem Ulcer Index gemäss Chaumontet et al., Arzneimittelforschung 1978, 28, 2047-2178 gemessen.
Tabelle 1 gibt die Ergebnisse wieder.

Tabelle 1

| Substanz | Ulcer Index (U.I.) | Anzahl Ratten |
|---|---|---|
| Vergl. 1% CMC 1 ml/250 g | 63,00 | 10 |
| Vergl. ASA 200 mg•kg$^{-1}$ | 300,00 | 20 |
| Erfi. ASC 200 mg•kg$^{-1}$ | 170,00 | 10 |
| Erfi. ASC 500 mg•kg$^{-1}$ | 190,00 | 10 |
| Erfi. ASC 1000 mg•kg$^{-1}$ | 220,00 | 10 |

CMC = Carboxymethylcellulose
ASA = Acetylsalicylsäure
SC = Acetylsalicoyl-L-Carnitin•HCl
Vergl. = Vergleich
Erfi. = Erfindung

## Patentansprüche

1. 3-(2-Acetoxybenzoyloxy)-4-(trimethylammonio)-buttersäurebetain

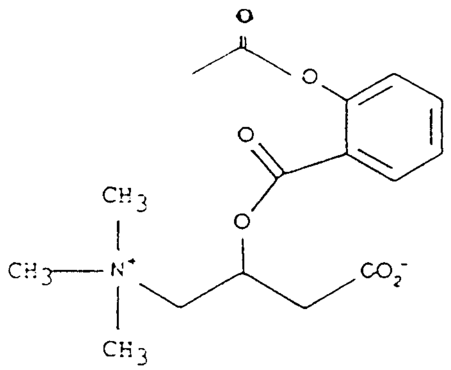

I

und dessen pharmazeutisch akzeptable Salze.

2. (R)-(-)-3-(2-Acetoxybenzoyloxy)-4-(trimethylammonio)-buttersäurebetain

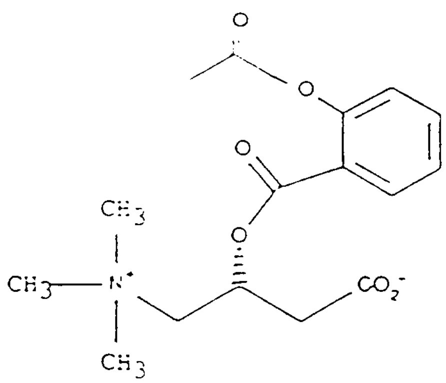

II

und dessen pharmazeutisch akzeptable Salze als Verbindungen nach Patentanspruch 1.

3. (S)-( + )-3-(2-Acetoxybenzoyloxy)-4-(trimethylammonio)-buttersäurebetain

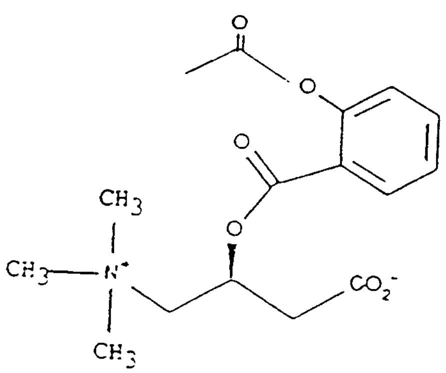

III

und dessen pharmazeutisch akzeptable Salze als Verbindungen nach Patentanspruch 1.

4. 3-(2-Acetoxybenzoyloxy)-4-(trimethylammonio)-buttersäure betain und dessen pharmazeutisch akzeptable Salze zur Anwendung als therapeutische Wirkstoffe.

5. (R)-(-)-3-(2-Acetoxybenzoyloxy)-4-(trimethylammonio)-buttersäurebetain und dessen pharmazeutisch akzeptable Salze zur Anwendung als thearpeutische Wirkstoffe.

6. (S)-(+)-3-(2-Acetoxybenzoyloxy)-4-(trimethylammonio)-buttersäurebetainund dessen pharmazeutisch akzeptable Salze zur Anwendung als therapeutische Wirkstoffe.

7. 3-(2-Acetoxybenzoyloxy)-4-(trimethylammonio)-buttersäurebetain und dessen pharmazeutisch akzeptable Salze zur Anwendung als die Magenschleimhaut schonende Analgetika.

8. (R)-(-)-3-(2-Acetoxybenzoyloxy)-4-(trimethylammonio)-buttersäurebetain und dessen pharmazeutisch akzeptable Salze zur Anwendung als die Magenschleimhaut schonende Analgetika.

9. (S)-(+)-3-(2-Acetoxybenzoyloxy)-4-(trimethylammonio)-buttersäurebetainund dessen pharmazeutisch akzeptable Salze zur Anwendung als die Magenschleimhaut schonende Analgetika.

10. Verfahren zur Herstellung von 3-(2-Acetoxybenzoyloxy)-4-(trimethylammonio)-buttersäurebetain, dadurch gekennzeichnet, dass ein 3-(2-Hydroxybenzoyloxy)-4-(trimethylammonio)buttersäurebetain acetyliert wird.

11. Verfahren nach Patentanspruch 10, dadurch gekennzeichnet, daß als Acetylierungsagens Acetylchlorid oder Essigsäureanhydrid in Gegenwart katalytischer Mengen $H_2SO_4$ verwendet werden.

12. Verfahren nach Patentanspruch 10 oder 11, dadurch gekennzeichnet, dass die Acetylierung bei Temperaturen zwischen 40 °C und 100 °C durchgeführt wird.

## Claims

1. 3-(2-Acetoxybenzoyloxy)-4-(trimethylammonio)-butyric acid betaine

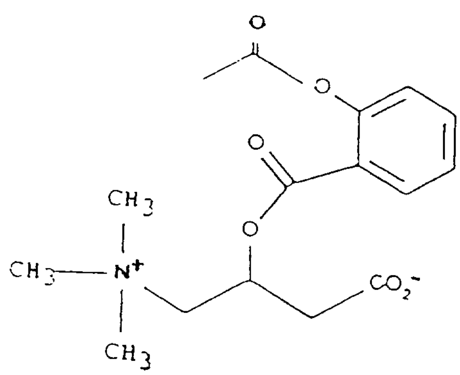

I

and pharmaceutically acceptable salts thereof.

**2.** (R)-(-)-3-(2-Acetoxybenzoyloxy)-4-(trimethylammonio)-butyric acid betaine

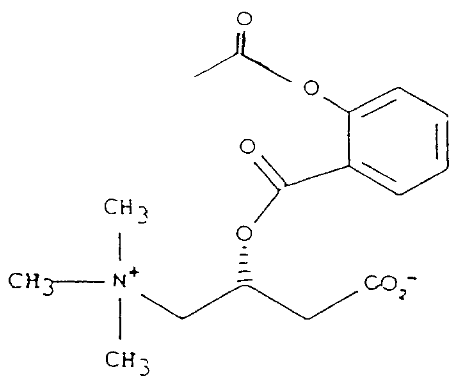

II

and pharmaceutically acceptable salts thereof as compounds according to claim 1.

**3.** (S)-( + )-3-(2-Acetoxybenzoyloxy)-4-(trimethylammonio)-butyric acid betaine

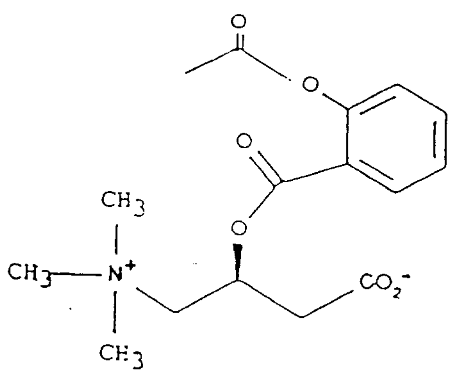

III

and pharmaceutically acceptable salts thereof as compounds according to claim 1.

**4.** 3-(2-Acetoxybenzoyloxy)-4-(trimethylammonio)-butyric acid betaine and pharmaceutically acceptable salts thereof for use as therapeutically active substances.

**5.** (R)-(-)-3-(2-Acetoxybenzoyloxy)-4-(trimethylammonio)-butyric acid betaine and pharmaceutically acceptable salts thereof for use as therapeutically active substances.

**6.** (S)-( + )-3-(2-Acetoxybenzoyloxy)-4-(trimethylammonio)-butyric acid betaine and pharmaceutically acceptable salts thereof for use as therapeutically active substances.

**7.** 3-(2-Acetoxybenzoyloxy)-4-(trimethylammonio)-butyric acid betaine and pharmaceutically acceptable salts thereof for use as analgetics gentle to the gastric mucosa.

**8.** (R)-(-)-3-(2-Acetoxybenzoyloxy)-4-(trimethylammonio)-butyric acid betaine and pharmaceutically acceptable salts thereof for use as analgetics gentle to the gastric mucosa.

**9.** (S)-( + )-3-(2-Acetoxybenzoyloxy)-4-(trimethylammonio)-butyric acid betaine and pharmaceutically acceptable salts thereof for use as analgetics gentle to the gastric mucosa.

**10.** Process for preparing 3-(2-acetoxybenzoyloxy)-4-(trimethylammonio)-butyric acid betaine, characterized in that a 3-(2-hydroxybenzoyloxy)-4-(trimethylammonio)-butyric acidbetaine is acetylated.

11. Process according to claim 10, characterized in that acetyl chloride or acetic anhydride are used as acetylating agents in the presence of catalytical amounts of $H_2SO_4$.

12. Process according to claims 10 or 11, characterized in that acetylation is carried out at temperatures of from 40 ° C and 100 ° C.

**Revendications**

1. Bétaïne de l'acide 3-(2-acétoxybenzoyloxy)-4-(triméthylammonio)-butyrique

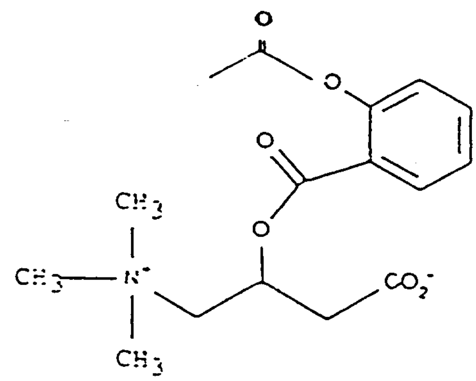

I

et ses sels pharmaceutiquement acceptables.

2. Bétaïne de l'acide (R)-(-)-3-(2-acétoxybenzoyloxy)-4-(triméthylammonio)-butyrique

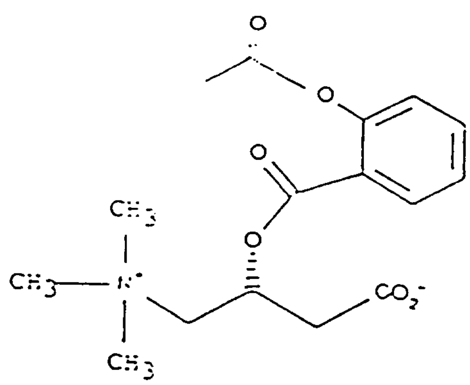

II

et ses sels pharmaceutiquement acceptables en tant que composés selon la revendication 1.

3. Bétaïne de l'acide (S)-(+)-3-(2-acétoxybenzoyloxy)-4-(triméthylammonio)-butyrique

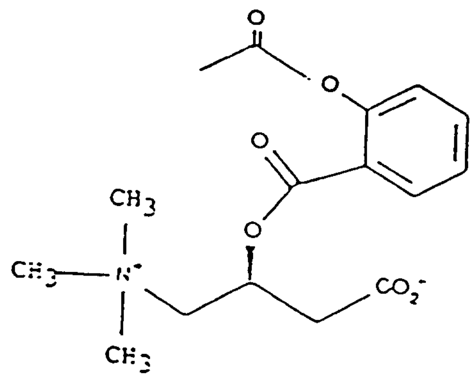

III

et ses sels pharmaceutiquement acceptables en tant que composés selon la revendication 1.

4. Bétaïne de l'acide 3-(2-acétoxybenzoyloxy)-4-(triméthylammonio)-butyrique et ses sels pharmaceutiquement acceptables pour l'utilisation en tant que substances thérapeutiques.

5. Bétaïne de l'acide (R)-(-)-3(2-acétoxybenzoyloxy)-4-(triméthylammonio)-butyrique et ses sels pharmaceutiquement acceptables pour l'application en tant que substances thérapeutiques.

6. Bétaïne de l'acide (S)-(+)-3-(2-acétoxybenzoyloxy)-4-(triméthylammonio)-butyrique et ses sels pharmaceutiquement acceptables pour l'application en tant que substances thérapeutiques.

7. Bétaïne de l'acide 3-(2-acétoxybenzoyloxy)-4-(triméthylammonio)-butyrique et ses sels pharmaceutiquement acceptables pour l'application comme analgésique ménageant les muqueuses stomacales.

8. Bétaïne de l'acide (R)-(-)-3-(2-acétoxybenzoyloxy)-4-(triméthylammonio)-butyrique et ses sels pharmaceutiquement acceptables pour l'application en tant qu'analgésique ménageant les muqueuses stomacales.

9. Bétaïne de l'acide (S)-(+)-3-(2-acétoxybenzoyloxy)-4-(triméthylammonio)-butyrique et ses sels pharmaceutiquement acceptables pour l'application en tant qu'analgésique ménageant les muqueuses stomacales.

10. Procédé pour la préparation de bétaïne de l'acide 3-(2-acétoxybenzoyloxy)-4-(triméthylammonio)-butyrique, caractérisé en ce que l'on acétyle une bétaïne de l'acide 3-(2-hydroxybenzoyloxy)-4-(triméthylammonio)butyrique.

11. Procédé selon la revendication 10, caractérisé en ce qu'en tant qu'agent d'acétylation, on utilise un chlorure d'acétyle ou un anhydride de l'acide acétique en présence de quantité catalytique d'$H_2SO_4$.

12. Procédé selon la revendication 10 ou 11, caractérisé en ce que l'acétylation est conduite à des températures entre 40 °C et 100 °C.